Europäisches Patentamt

European Patent Office

Office européen des brevets

(19)

(11) Publication number: **0 248 979**
**B1**

(12)

# EUROPEAN PATENT SPECIFICATION

(45) Date of publication of the patent specification:
20.06.90

(21) Application number: 87102859.3

(22) Date of filing: 27.02.87

(51) Int. Cl.⁵: **F16L 37/08, F16L 37/12,**
**A61M 5/00, A61M 1/14,**
**A61M 25/00**

(54) Coupling component.

(30) Priority: 06.06.86 SE 8602562

(43) Date of publication of application:
16.12.87 Bulletin 87/51

(45) Publication of the grant of the patent:
20.06.90 Bulletin 90/25

(84) Designated Contracting States:
BE CH DE ES FR GB IT LI NL

(56) References cited:
EP-A- 0 217 055
DE-A- 2 634 367
US-A- 4 369 781
US-A- 4 439 188
US-A- 4 452 473

(73) Proprietor: GAMBRO AB, Post Box 10101,
S-220 10 Lund(SE)

(72) Inventor: Carlsson, Per-Olov, Morkullevägen 11,
S-280 10 Sösdala(SE)

(74) Representative: Boberg, Nils Gunnar Erik, Gambro AB
Patent Department Box 10101, S-220 10 Lund(SE)

ACTORUM AG

## Description

### TECHNICAL FIELD

The present invention relates to a coupling component comprising a female part intended to be coupled together with a corresponding coupling component in the form of a male part, comprising an inner flexible cylindrical first coupling part which at its one end is provided with an orifice portion, which is intended to enclose a component of the male part with a friction grip, whilst it is provided at its other end with means for connection to a tube, bag or the like, and an outer torsionproof second coupling part which is connected to the inner part in such a manner that the two parts can be made to move together axially. Such couplings are used often in medical context for the joining together, for example, of tubes or for the joining together of a tube with another component, e g a bag, a dialyzer, an oxygenator or the like. A coupling of this type is often called a Luer coupling or Luer-Lock coupling, if it is provided with means for the locking together of the two components, e g co-operating screw threads or parts of screw threads.

### BACKGROUND ART

For Luer-Lock couplings frequently relatively hard material is used so that the screw threads used as locking should hold. Thanks to this locking it is possible in spite of all that to obtain a relatively good seal between coupling components included.

In Luer couplings without locking frequently a relatively flexible material is used in the female part in order to ensure a satisfactory seal. A coupling of this kind is known through for instance US-A 4 369 781.

Because of what is mentioned above different female parts are often used in Luer couplings with locking and in those without locking, respectively. It is desirable, though, that the same female part should be usable in both types of couplings whilst achieving a satisfactory sealing and locking.

### DISCLOSURE OF INVENTION

The above mentioned problem is solved in accordance with the present invention in that said inner flexible cylindrical first coupling part consists of a separate part made of relatively soft material and that said outer torsion-proof second coupling part consists of a separate part made of a relatively harder material. Thanks to this design a supple and effective seal can be achieved between the male part and the female part at the same time as the locking component of the female part, e g a screw thread, may be formed in the outer coupling part, which thus can be manufactured from a harder material.

In order to prevent any mutual rotation between the two parts, the outer part may be provided with one or more holes and/or slots, into which engage components projecting from the inner part. Such locking is achieved preferably at two or more points at a distance from each other seen in the longitudinal direction of the parts.

The outer part is preferably provided with an external thread or part of a thread which is intended to co-operate with a corresponding thread portion on the male part for the pulling together of the two coupling components. This external thread, owing to the invention, can be made of relatively hard material, at the same time as the seal towards the male part is achieved with the help of the more flexible material of the inner part. A further advantage, especially in a medical context, is that the outer part does not come into contact with the flow passing through the coupling component. Consequently it can be manufactured from a cheaper material and can be painted without the flow thereby being affected.

In a preferred embodiment of the subject of the invention the outer part is provided at its end facing towards the male part with a hook, flange or the like, which is adapted to compress the inner part axially on decoupling. This facilitates the decoupling, since the axial compression achieves an increase of the inner diameter.

The inner part, at least in the portion adjoining the said hook, flange or the like, is suitably arranged in the outer part with a clearance between the two parts so as to facilitate the said compression.

In order to facilitate coupling together, the said orifice portion enclosing the male part may be of a conical design and fit to the male part so that the latter on introduction brings about an axial compression of the inner part.

The coupling and decoupling are faciliated also if the outer part is designed with griping surfaces suitable for traction, compression as well as torsion.

The inner part preferably is fixed in the outer in such a manner that any traction in the said tube, bag or the like produces an axial stretching of the inner part and in particular in the orifice portion of the same enclosing the male part, which as a result is made further to strengthen its grip on the male part.

Special tool engineering advantages are obtained if the outer torsion-proof second cupling part is joined to the inner flexible cylindrical first coupling part in such a manner that mutual rotation between the two parts is prevented. Through this the wear of the inner part too will be reduced.

### BRIEF DESCRIPTION OF THE DRAWINGS

The invention is described in more detail in the following with reference to the attached drawings.

Fig 1 shows a female part in accordance with the invention together with a corresponding male part before coupling together.

Fig 2 shows the same components during coupling.

Fig 3 shows the components coupled together.

Fig 4 shows the same components during decoupling.

Fig 5, 6, 7 and 8 show examples of different male coupling components without special locking components which suitably can be used together with the female part in accordance with the present invention.

Fig 9-16 show in European representation different elevations and sections of a preferred embodiment of an outer part included in the coupling component in accordance with the invention.

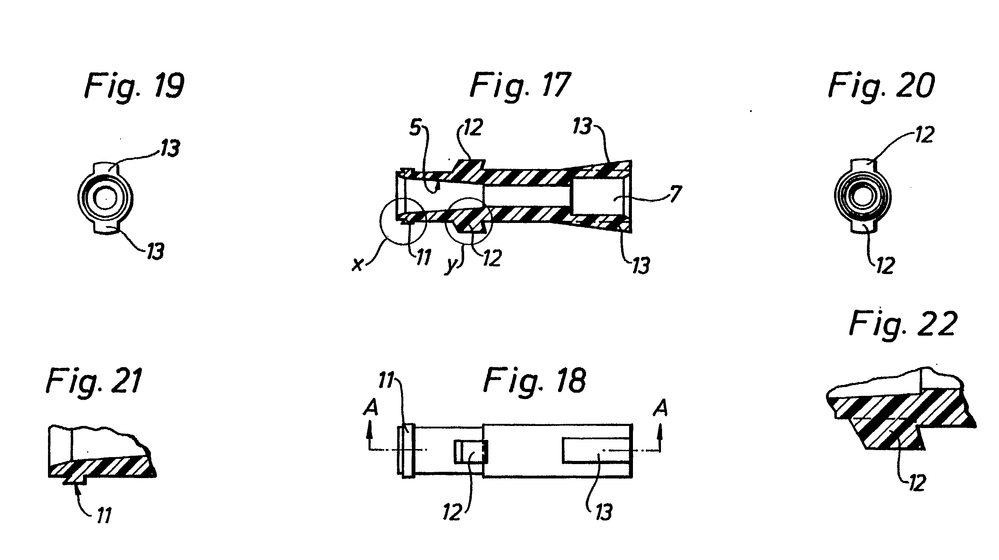

In the same manner Fig 17-22 show different elevations and sections of a preferred embodiment of an inner part included in the coupling component in accordance with the invention.

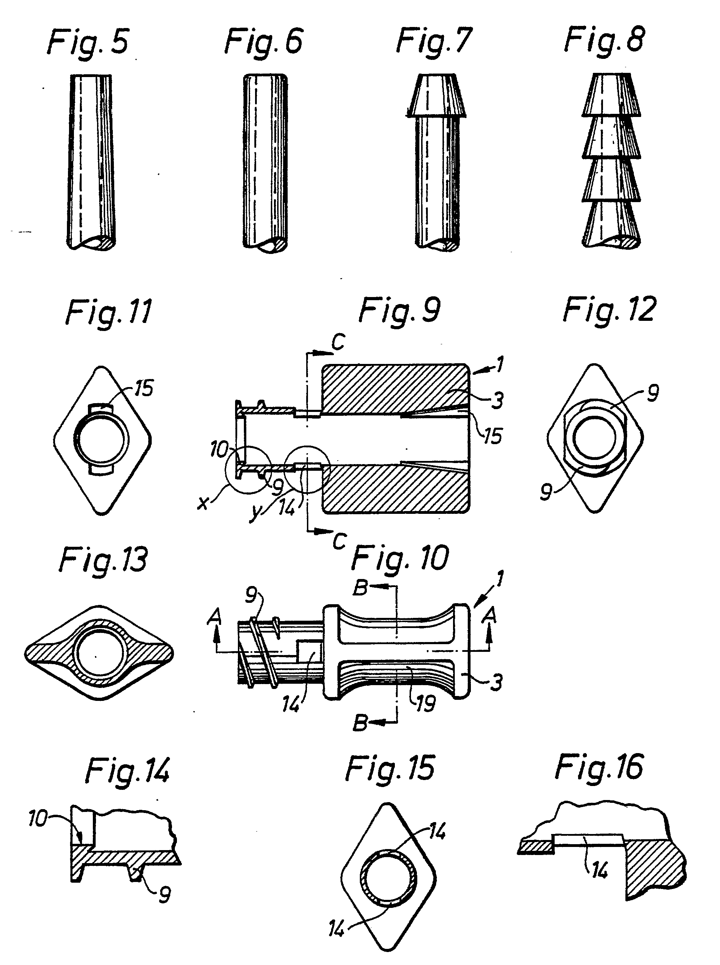

More particularly Fig 9 shows a section along line A-A in fig 10. Fig 10 shows the part according to Fig 9 seen from the top. Fig 11 shows the part according to Fig 9 seen from the right. Fig 12 shows the part according to Fig 9 seen from the left. Fig 13 shows a section along line B-B in Fig 10. Fig 14 shows the detail marked x in Fig 9 on a larger scale. Fig 15 shows a section along line C-C in Fig 9. Fig 16 shows the detail marked y in Fig 9 on a larger scale.

In the same manner Fig 17 shows a section along line A-A in Fig 18. Fig 18 shows the part according to Fig 17 seen from the top. Fig 19 shows the part according to Fig 17 seen from the right. Fig 20 shows the part according to fig 17 seen from the left. Fig 21 shows the part marked x in Fig 17 on a larger scale. Fig 22 finally shows the part marked y in Fig 17 on a larger scale.

## DETAIL DESCRIPTION OF THE INVENTION

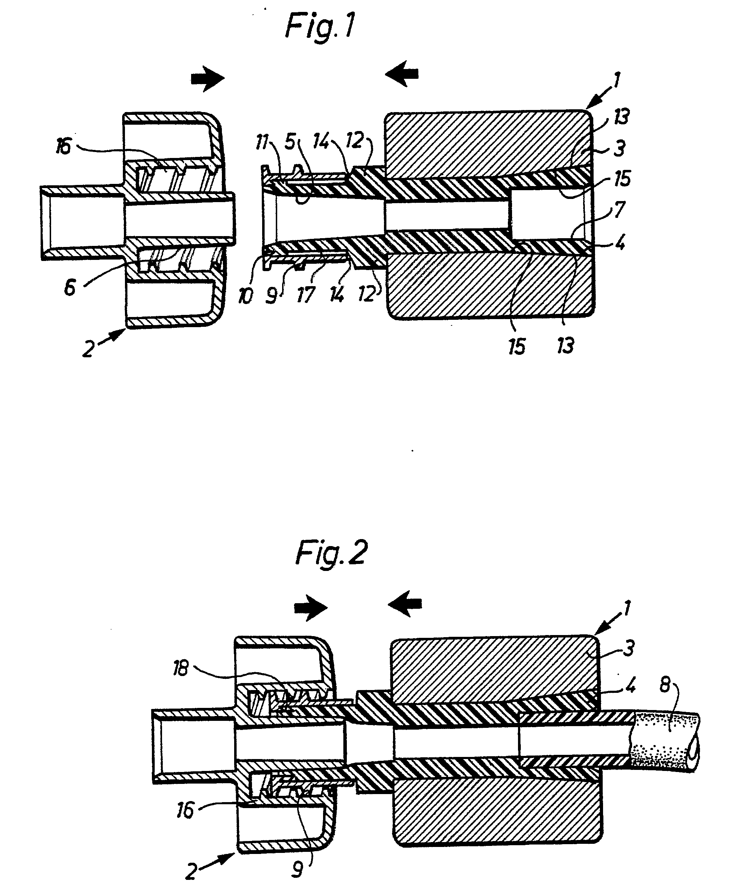

Fig 1-4 are intended to illustrate schematically the principles behind the present invention. In these figures is thus shown a female part 1 according to the invention together with a suitable male part 2 matching it. The female part 1 consists of an outer torsion-proof part 3 of a harder material and an inner flexible part 4 of a softer material. At its front end facing towards the male part 2 the female part 1 is provided with an inner, conical orifice portion 5 which is intended to enclose with a friction grip a corresponding coupling portion 6 on the male part 2. At its other end the inner part 4 is provided with a recess 7 intended to serve as a socket for a tube 8. Right in front of the orifice portion 5 of the inner part 4 the outer part 3 is provided with a screw thread 9. Furthermore it is provided inside of this thread with an internal flange 10 which is shown on a larger scale in Fig 14 and which co-operates with an external flange 11 on the inner part 4 which in turn is shown on a larger scale in Fig 21. In order to prevent torsion between the outer part 3 and the inner part 4 the latter is provided with two projecting wings 12 and two flanges 13. These components co-operate with holes 14 and slots 15 respectively in the outer part 3, whose detailed design can best be seen from Fig 9-16.

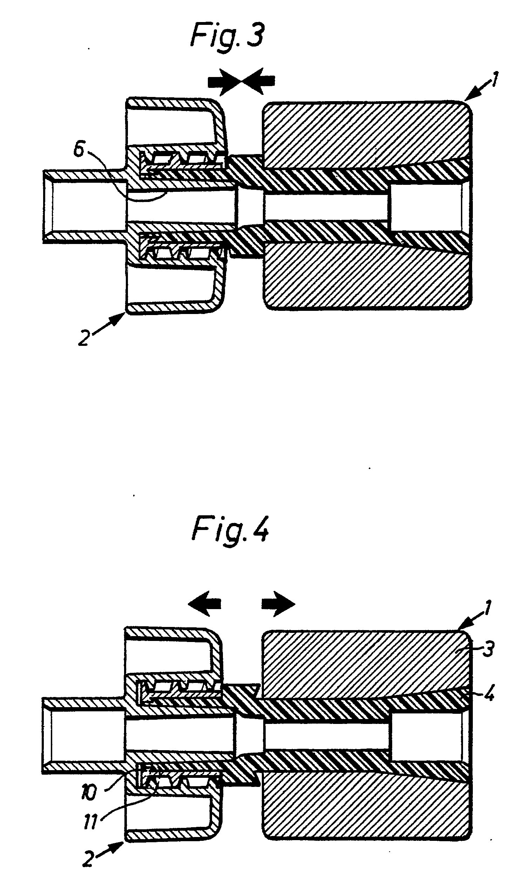

The coupling together of the female part 1 with the male part 2 is illustrated in Fig 2. The two parts are pulled together with the help of the screw thread 9 on the female part and the screw thread 16 on the male part. In so doing the inner part 4 is axially compressed, so that the clearance 17 shown in Fig 1 wholly or partly disappears. At the same time a smaller clearance 18 is created between the hooklike flanges 10 and 11. Owing to the compression, the orifice portion 15 is slightly expanded, thereby facilitating the coupling together. In Fig 3 are shown the two coupling components fully joined together. Because of the friction forces the greater part of the axial compression persists. If it slackens slightly, the friction grip around the component 6 on the male part 2 increases instead.

In Fig 4 a decoupling is illustrated. When the outer part 3 is twisted for this decoupling, the hooklike components 10 and 11 engage with one another and an axial compression of the inner part 4 is achieved, which causes the inner diameter of the orifice portion 5 slightly to increase, so that the moving asunder is facilitated.

If instead a traction occurs in the tube 8, an axial stretching of the inner part 4 is produced, especially in Luer couplings without screw thread locking, which brings about an increase in the friction grip between the component 6 and the orifice portion 5 enclosing it.

## BEST MODE OF CARRYING OUT THE INVENTION

In Fig 9-16 is shown a preferred embodiment of the outer part of a coupling component in accordance with the invention. In the same manner in Fig 17-22 is shown a preferred embodiment of the inner part of the same coupling component. Since the embodiment substantially agrees with that according to Fig 1-4 the same reference designations have been used. In Fig 9-22, however, the two parts are shown in greater detail, special attention being drawn to the following components.

As is evident from Fig 9, the slots 15 are angled. This facilitates the insertion of the inner part 4 with its wings 12. After the insertion, the flanges 13, shown for example in Fig 18, fill the slots 15 completely. The screw thread 9 and the hole 14 respectively, inside the rings marked x and y in Fig 9, are shown on a larger scale in Fig 14 and 16 respectively. In Fig 14 the hooklike flange 10 too is evident.

In Fig 10 is shown how the outer part 3 has been designed with well-shaped gripping surfaces 19 suitable for compression, traction as well as torsion.

The components marked x and y in Fig 17 are shown on a larger scale in fig 21 and Fig 22 respectively. Here the hooked flange 11 and one of the wings 12 too are evident. These components are also shown clearly in Fig 18. The design of the flanges 13 is evident best when Fig 17 and 18 are compared. Since the components according to fig 9-22 substantially function in the same manner as those according to Fig 1-4, no further description of them should be required.

Fig 5-8 finally show as an example four different male couplings which may be used advantageously with the female part according to the invention, and this without any special screw thread locking. On coupling with these components the soft inner part 4 behaves essentially in the same manner as on coupling with the component 6 shown in Fig 1-4. The same applies to decoupling.

## Claims

1. A coupling component comprising a female part (1) intended to be coupled together with a corresponding coupling component in the form of a male part (2), comprising an inner flexible cylindrical first coupling part (4) which at its one end is provided with an orifice portion (5), which is intended to enclose a component (6) of the male part with a friction grip, whilst it is provided at its other end with means (7) for connection to a tube (8), bag or the like, and an outer torsionproof second coupling part (3) which is connected to the inner part in such a manner that the two parts can be made to move together axially, characterized in that said inner flexible cylindrical first coupling part (4) consists of a separate part made of a relatively soft material and that said outer torsionproof second coupling part consists of a separate part made of a relatively harder material.

2. A coupling component in accordance with claim 1, characterized in that the outer part (3) is provided with one or more holes (14) and/or slots (15) into which engage components (12, 13) projecting from the inner part (4).

3. A coupling component in accordance with any one of claims 1 and 2, characterized in that the outer part (3) is provided with an external thread (9) or part of a thread which is intended to co-operate with a corresponding thread portion (16) on the male part (2) for the pulling together of the two coupling components.

4. A coupling component in accordance with any one of the preceding claims, characterized in that the outer part (3) on its end facing the male part (2) is provided with a hook flange or the like (10) engaging in the inner part, which is adapted to compress the inner part (4) axially on decoupling.

5. A coupling component in accordance with claim 4, characterized in that the inner part (4), at least in the portion nearest the said hook, flange or the like (10) is arranged in the outer part with a clearance (17) between the two parts in order to facilitate the said compression.

6. A coupling component in accordance with any one of the preceding claims, characterized in that the orifice portion (5) enclosing the said component (6) of the male part (2) is conical and fits to the male part so that the latter on introduction brings about an axial compression of the inner part.

7. A coupling component in accordance with any one of the preceding claims, characterized in that the outer part (3) is designed with gripping surfaces (19) suitable for traction, compression as well as torsion.

8. A coupling component in accordance with any one of the preceding claims, characterized, in that the inner part (4) is fixed in the outer (3) in such a manner that a traction in the said tube (8), bag or the like brings about an axial stretching of the inner part (4), especially in the orifice portion (5) of the same enclosing a component (6) of the male part.

9. A coupling component in accordance with any one of the preceding claims, characterized in that the outer torsion-proof second coupling part (3) is connected to the inner flexible cylindrical first coupling part (4) in such a manner that mutual rotation is prevented between the two parts.

## Patentansprüche

1. Kupplungsteil mit einem Aufnahmeteil (1), welches dafür vorgesehen ist, mit einem entsprechenden Kupplungsteil in der Form eines Einsteckteiles (2) zusammengekuppelt zu werden, mit einem inneren flexiblen zylindrischen ersten Kupplungsteil (4), welches an seinem einen Ende mit einem Öffnungsteil (5) versehen ist, welches dafür vorgesehen ist, ein Teil (6) des Einsteckteils mit einem Friktionsgriff einzuschließen, während es an seinem anderen Ende mit Mitteln (7) zum Verbinden mit einem Rohr, Schlauch (8), Beutel oder dergleichen und einem äußeren verdrehungssteifen zweiten Kupplungsteil (3) versehen ist, welches mit dem inneren Teil in solcher Weise verbunden ist, daß die zwei Teile zur axialen Bewegung zusammen veranlaßt werden können, dadurch gekennzeichnet, daß das innere flexible zylindrische erste Kupplungsteil (4) aus einem separaten Teil besteht, welches aus einem relativ weichen Material hergestellt ist, und daß das äußere verdrehungssteife zweite Kupplungsteil aus einem separaten Teil besteht, welches aus einem relativ härteren Material hergestellt ist.

2. Kupplungsteil nach Anspruch 1, dadurch gekennzeichnet, daß das äußere Teil (3) mit einem oder mehreren Löchern (14) und/oder Schlitzen (15) versehen ist, in welche aus dem inneren Teil (4) hervorstehende Teile (12, 13) in Eingriff treten.

3. Kupplungsteil nach einem der Ansprüche 1 und 2, dadurch gekennzeichnet, daß das äußere Teil (3) mit einem Außengewinde (9) oder einem Teil eines Gewindes versehen ist, welches dafür vorgesehen ist, mit einem entsprechenden Gewindeteil (16) auf dem Einsteckteil (2) zum Zusammenschieben der zwei Kupplungsteile zusammenzuwirken.

4. Kupplungsteil nach einem der vorhergehenden Ansprüche, dadurch gekennzeichnet, daß das äußere Teil (3) an seinem dem Einsteckteil (2) zugewandten Ende mit einem Hakenflansch oder dergleichen (10) versehen ist, welcher in dem inneren Teil in Eingriff tritt, welches geeignet derart ausgestaltet ist, daß es beim Entkuppeln das innere Teil (4) axial zusammendrückt.

5. Kupplungsteil nach Anspruch 4, dadurch gekennzeichnet, daß das innere Teil (4), mindestens in dem Teil am nächsten dem Haken, Flansch oder dergleichen (10), in dem äußeren Teil mit einem Spiel (17) zwischen den zwei Teilen angeordnet ist, um das Zusammendrücken zu erleichtern.

6. Kupplungsteil nach einem der vorhergehenden Ansprüche, dadurch gekennzeichnet, daß das Öffnungsteil (5), welches den Bestandteil (6) des Einsteckteils (2) einschließt, konisch ist und zu dem Einsteckteil derart paßt, daß letzteres beim Einführen eine axiale Kompression des inneren Teils zustandebringt.

7. Kupplungsteil nach einem der vorhergehenden Ansprüche, dadurch gekennzeichnet, daß das äußere Teil (3) mit Griffoberflächen (19) ausgestaltet ist, die für ein Ziehen, Komprimieren sowie Tordieren geeignet sind.

8. Kupplungsteil nach einem der vorhergehenden Ansprüche, dadurch gekennzeichnet, daß das innere Teil (4) in dem äußeren (3) in einer solchen Weise befestigt ist, daß ein mechanischer Zug in dem Schlauch (8), Beutel oder dergleichen ein axiales Strecken des inneren Teils (4) zustandebringt, insbesondere in dem Öffnungsteil (5) desselben, welches ein Teil (6) des Einsteckteiles einschließt.

9. Kupplungsteil nach einem der vorhergehenden Ansprüche, dadurch gekennzeichnet, daß das äußere verdrehungssteife zweite Kupplungsteil (3) mit dem inneren flexiblen zylindrischen ersten Kupplungsteil (4) derart verbunden ist, daß eine gegenseitige Drehung zwischen den zwei Teilen verhindert ist.

**Revendications**

1. Composant d'accouplement comportant une partie femelle (1) destinée à être accouplée à un composant d'accouplement correspondant sous la forme d'une partie mâle (2), comprenant un premier élément d'accouplement cylindrique flexible intérieur (4) qui présente à une extrémité une région d'orifice (5), prévue pour enserrer un élément (6) de la partie mâle avec prise par friction, tandis qu'il comporte à son autre extrémité des moyens (7) de jonction à un tube (8), un sac ou analogue, et un deuxième élément d'accouplement extérieur résistant à la torsion (3) qui est connecté à l'élément intérieur d'une manière telle que les deux éléments peuvent être déplacés ensemble axialement, caractérisé en ce que ledit premier élément d'accouplement cylindrique flexible intérieur (4) est constitué d'une pièce distincte en matière relativement tendre et en ce que ledit deuxième élément d'accouplement extérieur résistant à la torsion est constitué d'une pièce distincte en matière relativement plus dure.

2. Composant d'accouplement suivant la revendication 1, caractérisé en ce que l'élément extérieur (3) comporte un ou plusieurs trous (14) et/ou fentes (15), dans lesquels s'engagent des saillies (12, 13) de l'élément intérieur (4).

3. Composant d'accouplement suivant l'une quelconque des revendications 1 et 2, caractérisé en ce que l'élément extérieur (3) comporte un filetage extérieur (9), ou une partie d'un filetage, qui est prévu pour coopérer avec un filetage correspondant (16) de la partie mâle (2) afin de rapprocher les deux composants d'accouplement l'un de l'autre.

4. Composant d'accouplement suivant l'une quelconque des revendications précédentes, caractérisé en ce que l'élément extérieur (3) comporte, sur son extrémité en regard de la partie mâle (2), une collerette en forme de crochet ou analogue (10) venant en contact avec l'élément intérieur et capable de comprimer axialement l'élément intérieur (4) lors de la séparation.

5. Composant d'accouplement suivant la revendication 4, caractérisé en ce que l'élément intérieur (4), au moins dans la partie la plus proche dudit crochet, collerette ou analogue (10), est disposé dans l'élément extérieur avec un jeu (17) entre les deux éléments, afin de faciliter ladite compression.

6. Composant d'accouplement suivant l'une quelconque des revendications précédentes, caractérisé en ce que la région d'orifice (5) entourant ledit élément (6) de la partie mâle (2) est conique et s'ajuste à la partie mâle de sorte que cette dernière, lors de l'introduction, engendre une compression axiale de l'élément intérieur.

7. Composant d'accouplement suivant l'une quelconque des revendications précédentes, caractérisé en ce que l'élément extérieur (3) comporte des surfaces de préhension (19) appropriées pour une traction, une compression et une torsion.

8. Composant d'accouplement suivant l'une quelconque des revendications précédentes, caractérisé en ce que l'élément intérieur (4) est fixé dans l'élément extérieur (3) d'une manière telle qu'une traction sur ledit tube (8), sac ou analogue, engendre un étirement axial de l'élément intérieur (4), en particulier dans la région d'orifice (5) de ce dernier qui entoure un élément (6) de la partie mâle.

9. Composant d'accouplement suivant l'une quelconque des revendications précédentes, caractérisé en ce que le deuxième élément d'accouplement extérieur résistant à la torsion (3) est connecté au premier élément d'accouplement cylindrique flexible intérieur (4) d'une manière telle qu'une rotation relative entre les deux éléments est empêchée.

## Fig.1

## Fig.2

*Fig. 3*

*Fig. 4*

Fig. 5

Fig. 6

Fig. 7

Fig. 8

Fig. 11

Fig. 9

Fig. 12

15

C

1

3

15

10

x

9

y

14

C

9

9

Fig. 13

Fig. 10

B

9

1

A

A

14

19

3

B

Fig. 14

Fig. 15

Fig. 16

10

9

14

14

14

14

Fig. 19

Fig. 17

Fig. 20

Fig. 22

Fig. 21

Fig. 18

EP 0 248 979 B1